# EUROPÄISCHE PATENTSCHRIFT

(11) **EP 1 649 834 B1**
(45) Veröffentlichungstag und Bekanntmachung des Hinweises auf die Patenterteilung: **15.08.2007**
(21) Anmeldenummer: 05018550.3
(22) Anmeldetag: 26.08.2005
(51) Int. Cl.: A61F 2/28, A61F 2/78

(54) **Subkutanes, intramuskuläres Lager für ein starres transkutanes Implantat**
Subcutaneous, intra-muscular coupling for a rigid transcutaneous implant
Logement intramusculaire sous-cutané pour implant transcutané fixe

(30) Priorität: 25.10.2004 DE 102004052409
(43) Veröffentlichungstag der Anmeldung: 26.04.2006
(73) Patentinhaber: ESKA Implants GmbH & Co. KG, 23556 Lübeck (DE)
(72) Erfinder: Grundei, Hans, Dr.-Ing., 23558 Lübeck (DE)
(74) Vertreter: Fuchs

(56) Entgegenhaltungen:
- DE-A1- 10 040 590
- DE-B3- 10 247 397
- DE-U1-3202004 014 04

## Beschreibung

Die Erfindung betrifft ein subkutanes, intramuskuläres Lager für ein starres transkutanes Implantat, welches intrakorporal in einem Knochenstumpf verankerbar ist und welches ein Zwischenstück zwischen dem Implantat und einer daran ankoppelbaren extrakorporalen Koppelungseinrichtung aufweist.

Ein derartiges Lager ist bekannt aus der DE 100 40 590 A1. Das darin beschriebene Lager besteht aus einem flexiblen Material und es weist eine Tülle auf, die das Implantat distal fest umschließt, sowie eine intrakorporal anzuordnende Überwurfhülse in Form eines flexiblen Faltenbalges, der proximal mit einem angeformten Bund in abdichtender Weise mit der Tülle verbunden ist, derart, dass zwischen der Innenwandung des Faltenbalges und Außenwandung der Tülle ein Hohlraum einer Mindestbreite frei bleibt. Dabei ist distal am Faltenbalg ein flexibles Gitternetzwerk angeordnet, dem sich distalseitig ein weiteres Gitternetzwerk mit einem höheren E-Modul anschließt.

Mit diesem Lager wird das Ziel verfolgt, dass sich Weichteile gegenüber dem starren Implantat bewegen können, ohne dass die Durchbruchstelle im Körperstumpfteil einem erhöhten Risiko einer Entzündung ausgesetzt wird.

Wenn dieses bekannte Lager auch bereits in der Praxis erfolgreich eingesetzt wird, birgt es das Risiko, dass im Falle beispielsweise einer Reinigung der Durchtrittstelle des Implantates durch den Ober schenkelstumpf mit einer Kanüle durch das flexible Material, in den meisten Fällen Silikon, hindurchgestochen wird und eine Verkeimung stattfindet.

Um dem entgegenzuwirken, ist in der DE 102 47 397 B3 ein subkutanes, intramuskuläres Lager vorgeschlagen worden, das eine mit dem Zwischenstück fest verbundene starre Buchse derart aufweist, dass zwischen der Wandung der Buchse und dem Zwischenstück ein in Richtung intrakorporal geschlossener Ringraum ausgebildet ist, in den die extrakorporale Koppelungseinrichtung setzbar ist. Es weist einen auf die Außenwandung der Buchse aufgebrachten Schlauch aus flexiblem Material und auf den flexiblen Schlauch aufgebrachte metallische Wolle auf. Hiermit wird das Ziel verfolgt, dass die Sicherheit gegen eine Verkeimung der Durchtrittsstelle des Implantates und der angrenzenden Bereiche des Oberschenkelstumpfes deutlich erhöht wird und dass ein versehentliches Entfernen der Keimschranke verhindert wird.

Wenn die Sicherheit bei diesem Lager auch schon recht hoch angesiedelt ist, besteht der Wunsch, diese noch weiter zu erhöhen, um dem Patienten eine äußert unangenehme Prozedur im Falle einer Keimung zu ersparen.

Vor diesem Hintergrund ist es nun die Aufgabe der vorliegenden Erfindung, ein subkutanes intramuskuläres Lager so weiterzubilden, dass die Sicherheit gegen eine Verkeimung der Durchtrittsstelle des lmplantates und der angrenzenden Bereiche des Oberschenkelstumpfes nochmals deutlich erhöht wird.

Demgemäß wird vorgeschlagen, dass das Lager eine mit dem Zwischenstück fest verbundene oder einstückig mit diesem ausgebildete starre Buchse mit einem in Richtung intrakorporal geschlossenen Ankoppelungselement aufweist, an das die extrakorporale Koppelungseinrichtung koppelbar ist. Es ist eine offenmaschige, dreidimensionale Raumnetzstruktur auf der Außenwandung der Buchse unter Aussparung des distalen Bereiches einer Breite B vorgesehen. Das Lager weist darüber hinaus ein Adaptionsrohr auf, das in das Innere der Buchse greift und dort lösbar in einem Presssitz sitzt, in welches die ankoppelbare Koppelungseinrichtung setzbar ist. Das Adaptionsrohr verfügt zumindest an seiner Außenwandung über eine antibakterielle Wirkung.

Gegenüber den bekannten Lagern ist die Buchse vorliegend ein starres Element, das nicht etwa durch Injektionskanülen durchdrungen werden kann. Die Buchse ist entweder fest mit dem Zwischenstück verbunden oder einstückig mit diesem ausgebildet.

Die auf der Außenwandung der Buchse vorgesehene offenmaschige, dreidimensionale Raumnetzstruktur dient dazu, dass sich Bindegewebe darin einorganisieren kann und so eine Keimschranke ausbildet. Der distale Bereich bleibt dabei von der Raumnetzstruktur ausgespart, um so eine Bewegung des umgebenden Bindegewebes bei Ausgleichsbewegungen zu ermöglichen.

Eine hohe Keimsperre wird durch die Verwendung des Adaptionsrohres erzielt. Es ist wichtig zu erwähnen, dass dieses Adaptionsrohr lösbar im Inneren der Buchse in einem Presssitz sitzt. Nimmt die antibakterielle Wirkung seiner Außenwandung im Laufe der Zeit ab, so kann es nach Lösung des Presssitzes durch ein neues, "frisches" Adaptionsrohr ersetzt werden. Hierzu muss lediglich die extrakorporale Koppelungseinrichtung vom Zwischenstück abgekoppelt werden und das Adaptionsrohr herausgezogen werden.

In bevorzugter Weiterbildung besteht das Adaptionsrohr aus massivem Silber. Die antibakterielle Wirkung von Silber ist allgemein bekannt.

Gemäß einer anderen Ausführungsform besteht das Adaptionsrohr aus einem Material, welches auf seiner Außenwandung versilbert ist. Diese Ausführungsform ist kostengünstiger als die vorstehend erwähnte. Beispielsweise kann als Basismaterial eine Kobaltchrommolybdänlegierung Anwendung finden.

Alternativ kann das Adaptionsrohr aus einem Material bestehen, welches auf seiner Außenwandung mit Hydroxylapatit, Kalziumphosphat, Titan oder Plasmatitanspray beschichtet ist. Die vorerwähnten Beschichtungsmaterialien haben die Eigenschaft, dass Haut und Bindegewebe sich an die Außenwandung anlagern und so auf der Länge des Adaptionsrohres für eine zusätzliche keimhemmende Wirkung sorgen. Gleichwohl wächst das umgebende Bindegewebe nicht in die Oberfläche des Rohres hinein, sondern lagert sich lediglich an, so dass die Lösbarkeit des Adaptionsrohres zum Zwecke des Ersatzes weiterhin gewährleistet bleibt.

Alternativ kann das Adaptionsrohr gemäß einer noch weiteren Ausführungsform aus Polyurethan bestehen. Polyurethan wird in der Medizintechnik als antibakterielles Material eingesetzt.

Gemäß einer besonderen Ausführungsform ist vorgesehen, dass das Adaptionsrohr eine solche Länge aufweist, dass es mit seiner distalen Stirnkante auf einer Schulter aufsitzt, die an der extrakorporalen Koppelungseinrichtung ausgebildet ist, wenn diese am Ankoppelungselement angekoppelt ist. Hierdurch wird ein sauberer Übergang von der extrakorporalten Koppelungseinrichtung hin zum Zwischenstück erzielt und Verschmutzungen bzw. Verkeimungen des Inneren des Adaptionsrohres weitgehend verhindert.

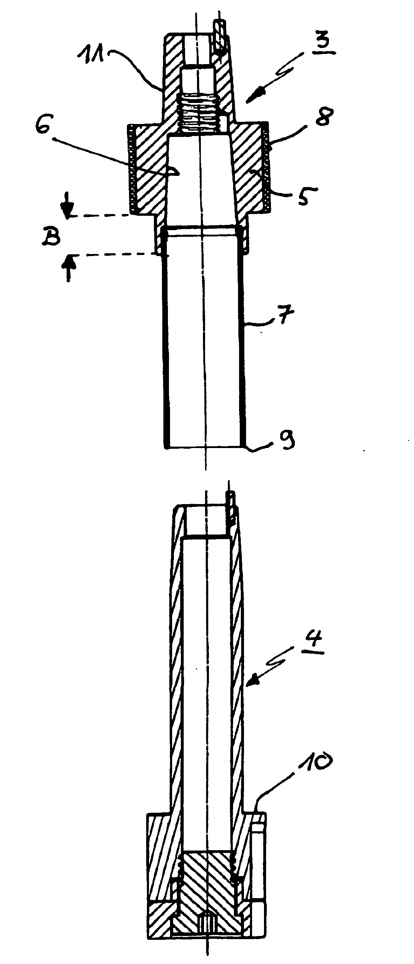

Die Erfindung wird anhand der einzigen Zeichnungsfigur beispielhaft näher erläutert. Diese zeigt im Schnitt das Zwischenstück mit angekoppeltem Adaptionsrohr sowie die extrokorporale Koppelungsvonichtung.

Das Zwischenstück 3 sitzt zwischen dem transkutanen Implantat (nicht dargestellt), mit welchem es über seinen Steckkonus 11 verbindbar ist.

Im dargestellten Ausführungsbeispiel ist mit dem Zwischenstück 3 die starre Buchse 5 einstückig ausgebildet. In ihrem Inneren ist ein in Richtung intrakorporal, in der Zeichnungsfigur also nach oben, geschlossenes Ankoppelungselement 6 vorgesehen, vorliegend als konische Klemmhülse angedeutet. An das Ankoppelungselement 6 ist die extrakorporale Koppelungseinrichtung 4 koppelbar.

Auf der Außenwandung der Buchse 5 ist eine offenmaschige, dreidimensionale Raumnetzstruktur 8 vorgesehen, und zwar unter Aussparung des distalen Bereiches der Breite B. In diese Raumnetzstruktur 8 wächst nach der Implantation Bindegewebe ein und bildet so eine weitere Keimschranke. Das Freilassen im distalen Bereich um die Breite B ermöglicht eine Bewegung des umgebenden Bindegewebes bei Ausgleichsbewegungen.

In das Innere der Buchse 5 greift das Adaptionsrohr 7. Es sitzt in dem Inneren der Buchse 5 in einem lösbaren Presssitz, damit es bei einem notwendigen Austausch entfernt und durch ein neues Adaptionsrohr ersetzt werden kann. In dieses Adaptionsrohr 7 ist die extrakorporale Koppelungseinrichtung 4 setzbar. Vorliegend sind die Dimensionen so gewählt, dass bei angekoppelter extrakorporaler Koppelungseinrichtung 4 die distale Stirnkante 9 des Adaptionsrohres 7 auf der Schulter 10, die an der Koppelungseinrichtung 4 ausgebildet ist, aufsitzt, wenn die Koppelungseinrichtung 4 an das Zwischenstück 3 angekoppelt ist. Hierdurch wird ein Formenschluss erzielt, der eine Verschmutzung des Inneren des Adaptionsrohres 7 unterbindet.

### Bezugszeichenliste

- 3: Zwischenstück
- 4: extrakorporale Koppelungseinrichtung
- 5: Buchse
- 6: Ankoppelungselement
- 7: Adaptionsrohr
- 8: Raumnetzstruktur
- 9: distale Stirnkante
- 10: Schulter
- **11**: **Steckkonus**

## Patentansprüche

1. Subkutanes, intramuskuläres Lager für ein starres transkutanes Implantat, welches intrakorporal in einem Knochenstumpf verankerbar ist, das ein Zwischenstück (3) zwischen dem Implantat und einer daran ankoppelbaren extrakorporalen Koppelungseinrichtung (4) aufweist, **gekennzeichnet durch**
- ein mit dem Zwischenstück (3) fest verbundene oder einstückig mit diesem ausgebildete starre Buchse (5) mit einem in Richtung intrakorporal geschlossenen Ankoppelungselement (6), an das die extrakorporale Koppelungseinrichtung (4) koppelbar ist,
- eine offenmaschige, dreidimensionale Raumnetzstruktur (8) auf der Außenwandung der Buchse (5) unter Aussparung des distalen Bereiches einer Breite B, und
- ein Adaptionsrohr (7), das in das Innere der Buchse (5) greift und dort lösbar in einem Presssitz sitzt, in welches die ankoppelbare Koppelungseinrichtung (4) setzbar ist, mit antibakterieller Wirkung zumindest an seiner Außenwandung.

2. Lager nach Anspruch 1, bei dem das Adaptionsrohr (7) aus Silber besteht.

3. Lager nach Anspruch 1, bei dem das Adaptionsrohr (7) aus einem Material besteht, welches auf seiner Außenwandung versilbert ist.

4. Lager nach Anspruch 1, bei dem das Adaptionsrohr (7) aus einem Material besteht, welches auf seiner Außenwandung mit Hydroxylapatit beschichtet ist.

5. Lager nach Anspruch 1, bei dem das Adaptionsrohr (7) aus einem Material besteht, welches auf seiner Außenwandung mit Kalziumphosphat beschichtet ist.

6. Lager nach Anspruch 1, bei dem das Adaptionsrohr (7) aus einem Material besteht, welches auf seiner Außenwandung mit Titan beschichtet ist.

7. Lager nach Anspruch 1, bei dem das Adaptionsrohr (7) aus einem Material besteht, welches auf seiner Außenwandung mit Plasmatitanspray beschichtet ist.

8. Lager nach Anspruch 1, bei dem das Adaptionsrohr (7) aus Polyurethan besteht.

9. Lager nach einem der Ansprüche 1 bis 8, bei dem das Adaptionsrohr (7) eine solche Länge aufweist, dass es mit seiner distalen Stirnkante (9) auf einer Schulter (10) aufsitzt, die an der am Ankoppelungselement (6) angekoppelten extrakorporalen Koppelungseinrichtung (4) ausgebildet ist

## Claims

1. Subcutaneous, intramuscular bearing for a rigid transcutaneous implant, which can be anchored intracorporeally in a bone stump, which has an intermediate piece (3) between the implant and an extracorporeal coupling device (4) which can be coupled thereto, **characterised by**
- a rigid bushing (5) firmly connected to the intermediate piece (3) or designed to be integral with the latter, and having a coupling element (6) which is closed intracorporeally, and to which the extracorporeal coupling device (4) can be coupled,
- an open-mesh, three-dimensional spatial network structure (8) on the outer wall of the bushing (5) except for the distal region of width B, and
- an adaptor tube (7), which engages in the interior of the bushing (5) and sits there releasably in a press fit, into which the coupling device (4), which can be coupled, can be placed, with anti-bacterial effect at least on its outer wall.

2. Bearing according to claim 1, in which the adaptor tube (7) consists of silver.

3. Bearing according to claim 1, in which the adaptor tube (7) consists of a material which is silver-plated on its outer wall.

4. Bearing according to claim 1, in which the adaptor tube (7) consists of a material which is coated with hydroxylapatite on its outer wall.

5. Bearing according to claim 1, in which the adaptor tube (7) consists of a material which is coated with calcium phosphate on its outer wall.

6. Bearing according to claim 1, in which the adaptor tube (7) consists of a material which is coated with titanium on its outer wall.

7. Bearing according to claim 1, in which the adaptor tube (7) consists of a material which is coated with plasma titanium spray on its outer wall.

8. Bearing according to claim 1, in which the adaptor tube (7) consists of polyurethane.

9. Bearing according to one of claims 1 to 8, in which the adaptor tube (7) has such a length that it sits with its distal end-face edge (9) on a shoulder (10), which is formed on the extracorporeal coupling device (4) coupled to the coupling element (6).

## Revendications

1. Appui intramusculaire sous-cutané pour un implant transcutané rigide, qui peut s'ancrer sur un moignon par voie intracorporelle, présentant un raccord (3) entre l'implant et un dispositif de liaison extracorporel (4) qui se couple sur celui-ci, **caractérisé par**
- une prise rigide (5) solidement fixée sur ou faisant partie intégrante du raccord (3), comportant un élément de couplage (6) fermé en direction du corps, sur lequel peut se coupler l'élément de couplage extracorporel (4),
- une structure tridimensionnelle à maillage large (8) sur la face externe de la prise (5) avec une fenêtre de largeur B dans la zone distale, et
- un tube adaptateur (7) qui s'engage dans la prise (5) et se loge de manière amovible dans une loge de compression, dans lequel le dispositif de couplage (4) peut se coupler, avec une action antibactérienne au moins sur sa face externe.

2. Appui selon la revendication 1, dans lequel le tube adaptateur (7) est en argent.

3. Appui selon la revendication 1, dans lequel le tube adaptateur (7) est composé d'un matériau dont la face externe est plaquée en argent.

4. Appui selon la revendication 1, dans lequel le tube adaptateur (7) est composé d'un matériau dont la face externe est plaquée en hydroxylapatite.

5. Appui selon la revendication 1, dans lequel le tube adaptateur (7) est composé d'un matériau dont la face externe est plaquée en phosphate de calcium.

6. Appui selon la revendication 1, dans lequel le tube adaptateur (7) est composé d'un matériau dont la face externe est plaquée en titane.

7. Appui selon la revendication 1, dans lequel le tube adaptateur (7) est composé d'un matériau dont la face externe est plaquée d'un spray plasma de titane.

8. Appui selon la revendication 1, dans lequel le tube adaptateur (7) est composé de polyuréthane.

9. Appui selon l'une des revendications 1 à 8, dans lequel le tube adaptateur (7) est d'une longueur qui lui permet de s'appuyer par le bord du front distal (9) sur une épaule (10) présente sur le dispositif extracorporel de couplage (4) couplé sur l'élément de couplage (6).
